(19) Europäisches Patentamt
European Patent Office
Office européen des brevets

(11) **EP 0 719 149 B1**

(12) **FASCICULE DE BREVET EUROPEEN**

(45) Date de publication et mention de la délivrance du brevet: **31.07.2002 Bulletin 2002/31**

(21) Numéro de dépôt: **94926979.9**

(22) Date de dépôt: **13.09.1994**

(51) Int Cl.$^7$: **A61K 35/04** // A61K7/48, A61K7/06,(A61K35/04, 31:165, A61P31:10)

(86) Numéro de dépôt international: **PCT/FR94/01072**

(87) Numéro de publication internationale: **WO 95/07702 (23.03.1995 Gazette 1995/13)**

(54) **COMPOSITION DERMOCOSMETIQUE ANTIFONGIQUE**

FUNGIZIDE, HAUTKOSMETISHE ZUSAMMENSETZUNG

ANTI-FUNGICIDAL DERMATOCOSMETIC COMPOSITION

(84) Etats contractants désignés:
**AT BE CH DE DK ES FR GB GR IE IT LI LU MC NL PT SE**

(30) Priorité: **14.09.1993 FR 9310921**

(43) Date de publication de la demande: **03.07.1996 Bulletin 1996/27**

(73) Titulaire: **Pierre Fabre Dermo-Cosmetique 92100 Boulogne (FR)**

(72) Inventeurs:
- **FABRE, Pierre F-81106 Castres (FR)**
- **JEANJEAN, Michel F-31320 Castanet Tolosan (FR)**
- **LAGARDE, Isabelle F-31520 Ramonville (FR)**

(74) Mandataire: **Warcoin, Jacques et al Cabinet Régimbeau 20, rue de Chazelles 75847 Paris cedex 17 (FR)**

(56) Documents cités:
**US-A- 3 980 775**

- **CHEMICAL ABSTRACTS, vol. 112, no. 14, 2 Avril 1990, Columbus, Ohio, US; abstract no. 125255z, page 422 ; & JP,A,01 287 042 (FUJINAGA PHARMACEUTICAL CO., LTD.)**
- **CHEMICAL ABSTRACTS, vol. 107, no. 4, 27 Juillet 1987, Columbus, Ohio, US; abstract no. 28311d, K. TAKEUCHI ET AL. 'EFFECT OF SOLUBILIZERS ON THE ANTI-INFLAMMATORY PROPERTY OF GLYMESASON OINTMENT.' page 318 ; & YAKURI TO CHIRYO, vol.14, no.7, 1986 pages 4723 - 4728**
- **S. BUDAVARI ET AL. (ED'S) 'THE MERCK INDEX' 1989 , MERCK & CO., INC. , RAHWAY, N.J., U.S.A. * page 406, ref. 2597 ***

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

## Description

La présente invention se rapporte à de nouvelles compositions utiles en dermatologie et/ou en cosmétologie présentant une activité antifongique améliorée.

Les champignons et les levures parasites sont impliqués dans de nombreuses affections dermatologiques courantes, comme le psoriasis ou la dermite séborrhéique. Des troubles de la kératinisation, entraînant notamment des phénomènes de desquamation ou d'irritation locales, sont également associés à une prolifération anormale de tels germes.

En particulier, la levure Pityrosporum ovale (aussi appelée Malassezia furfur) est présente dans les pathologies des états pelliculaires et squameux.

Différents principes actifs antifongiques connus ont été proposés, parmi lesquels on peut citer la piroctonolamine, le sulfure de sélénium, le pyrithionne de zinc, l'acide salicylique, les undécylénates. Toutefois, aucune ne possède une activité optimale sur toutes les souches de P. ovale, en particulier les souches résistant aux imidazolés.

Les goudrons de diverses origines ont été utilisés en pratique dermatologique, sous différentes présentations. En raison de leur caractéristique olfactive marquée, ils sont, dans la majorité des cas, formulés dans des produits destinés à être rincés et leur temps de contact avec la peau et le cuir chevelu est limité ; en outre leur application n'est pas agréable pour l'utilisateur, ce qui constitue un inconvénient important pour des compositions d'hygiène et de cosmétologie.

Il existe donc un besoin pour un produit antifongique à large spectre, convenant pour des utilisations dans le domaine de la dermatologie et/ou de la cosmétologie.

C'est pourquoi la présente invention a pour objet une composition dermocosmétique, antifongique, caractérisée en ce qu'elle contient, outre un véhicule dermatologiquement acceptable, une association synergique de crotamiton et d'au moins un goudron à condition que ce goudron ne soit pas un goudron de soja.

En effet, la Demanderesse a trouvé que, de manière inattendue, l'activité antifongique du goudron est potentialisée dans des mélanges avec le crotamiton, ce qui permet de diminuer fortement les concentrations efficaces ; les compositions contenant de telles associations synergiques sont actives sur des souches généralement résistantes, en particulier aux imidazolés, comme l'éconazole, ces souches pathogènes étant relativement fréquentes.

La synergie peut être mise en évidence par le calcul des index FIC (Fractional Inhibitory Concentration) ou FBC (Fractional Bactericidal Concentration) des produits. Pour cela, on mesure la valeur de la CMF (ou concentration minimale fongistatique), ou de la $CM_{\varphi}$ (ou concentration minimale fongicide), des principes actifs à tester vis-à-vis d'un type de germe.

Le FIC d'un produit A est défini comme :

$$FIC_A = \frac{\text{CMF du produit A en association}}{\text{CMF du produit A seul}}$$

L'association de deux antifongiques A et B est synergique si la somme FIC des rapports ($FIC_A + FIC_B$) est inférieure ou égale à 0,75.

Plus la valeur FIC est faible, plus la synergie est importante.

On considère qu'il y a simple additivité pour des valeurs de FIC comprises entre 0,75 et 1,1, et indifférence dans l'intervalle compris entre 1,1 et 2 ; au-delà, l'association est notée antagoniste.

La représentation graphique des interactions a lieu à l'aide des isobologrammes.

Un isobologramme est représenté dans un repère orthonormé avec en abscisse et en ordonnées les CMF (ou $CM_{\varphi}$) des antifongiques en association.

L'isobole est la ligne qui joint les points représentant les associations inhibitrices ou fongicides y compris les points correspondant aux CMF ou $CM_{\varphi}$ des antifongiques seuls.

Les points représentant les différentes associations synergiques seront sur l'isobole droite, au-dessous de cette droite pour les associations synergiques et au-dessus pour les associations indifférentes ou antagonistes.

Le crotamiton, ou N-éthyl N-O-tolylcrotonamide a pour formule :

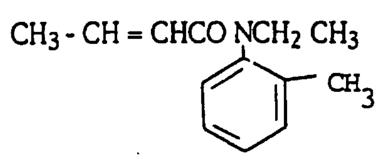

et a été décrit comme scabicide et antiprurigineux.

Les compositions selon la présente invention contiennent de préférence des associations binaires ou ternaires de crotamiton avec un ou plusieurs goudrons à condition que ce goudron ne soit pas un goudron de soja.

Par goudron, on entend un ensemble de composés dont la teneur en phénols, hydrocarbures cycliques, dérivés sulfurés et pyridiques peut varier en fonction de leur origine et de leur dégré de purification.

Les goudrons particulièrement appropriés pour les compositions selon l'invention comprennent :

- des dérivés de la houille, ce sont les coaltars à l'état "crude" ou modifiés,
- des dérivés végétaux issus de la pyrrolyse des bois comme par exemple les goudrons de pin, de cade ou de bouleau,
- des dérivés de schistes bitumineux comme l'ichthyol ammonium.

Par goudron brut, on entend le produit issu directement du cracking de la houille, par exemple, et renfermant toutes les fractions distillant entre 60 et 370° jusqu'au brai.

On y trouve donc des huiles légères jusqu'aux hydrocarbures lourds représentés par des huiles anthracéniques, puis les brais constitués par des éléments insolubles en suspension.

Généralement, ce sont les "Queues" de distillation et les brais qui renferment les structures polycycloaromatiques responsables du potentiel cancérigène des goudrons.

Le goudron "crude" ou brut pourra subir différentes modifications visant à le purifier ou l'alléger pour le rendre plus facile d'emploi à la formulation ou moins désagréable à l'utilisation.

Par goudron lavé, on entend goudrons débarrassé de tous ses insolubles. Cette extraction pouvant se faire par solvants de type méthylal, hexane, toluène, etc.

La purification du goudron peut être obtenue selon deux voies :

. par distillation du goudron lavé, les températures définissant alors le distillat obtenu. Ainsi, on peut passer de 9000 PPM de BENZO (a) PYRENE à 170 PPM.

. par extraction faisant appel à un solvant sélectif. C'est le cas du "coaltar solution" de l'USP obtenu par extraction à l'aide d'une solution de polysorbate 80 dans l'alcool.

Ces derniers constituent des formes plus acceptables et d'une mise en oeuvre plus aisée en raison de leur dispersibilité améliorée dans les bases dermatologiques.

De préférence, la concentration en goudron dans les compositions selon l'invention est située dans l'intervalle compris entre 0,5 et 5 g/100ml.

La concentration en crotamiton dans les compositions selon l'invention est de préférence située dans l'intervalle compris entre 0,25 et 5 g/100ml.

Des compositions présentant une synergie optimale sont obtenues quand le rapport des concentrations des composés de type goudron au crotamiton est compris entre 2 et 20, et de manière avantageuse entre 1 et 4.

Les concentrations respectives des composants, ainsi que le choix du ou des goudrons pourront être adaptés par l'homme du métier en fonction de l'application visée : dans des compositions pharmaceutiques, pouvant être destinées à lutter contre des infections à germes multirésistants, les doses pourront être augmentées, alors que les compositions d'hygiène et de cosmétologie privilégieront l'aspect et l'agrément d'application, tout en gardant une bonne efficacité.

De manière générale, alors que les goudrons seuls doivent être utilisés à une concentration de l'ordre de 6% (0,6 g/ml), cette concentration peut être diminuée à 2% et même moins dans les compositions selon l'invention.

Les compositions selon l'invention peuvent être formulées notamment en crème, lotion, shampooing, onguent, pain dermatologique. Des excipients appropriés, tels que parfums, stabilisants, tensioactifs, conservateurs, colorants sont ajoutés par des technologies connues de l'homme du métier.

Selon l'un des aspects de l'invention, les compositions antifongiques synergiques contiennent également des huiles essentielles, qui outre leur caractère aromatique, peuvent présenter une activité antiseptique intrinsèque renforçant l'activité de la composition.

Dans tous les cas, une synergie d'activité entre les goudrons et le crotamiton a été démontrée sur P. ovale, germe directement impliqué dans la pathologie des pellicules.

Les synergies sont efficaces sur les souches comportant une résistance aux imidazolés, qui ont une probabilité de fréquence élevée étant donné que ce type de molécule est utilisé depuis une vingtaine d'années.

En outre, les goudrons possèdent une activité réductrice et kératoplastique induisant, par inhibition de la mitose, une diminution numérique et dimensionnelle des cellules des couches superficielles épidermiques.

Par ailleurs, l'activité antiprurigineuse du crotamiton est particulièrement intéressante dans le traitement des pathologies tissulaires accompagnées de purit du type pellicules, états squameux, psoriasis, parasitoses, affections séborrhéïques, etc.

Enfin, le pouvoir solvant du crotamiton joue un rôle important comme facteur de pénétration cutanée.

Il est donc possible d'associer des actifs à doses plus faibles donc moins toxiques et plus acceptables.

Cette association présente donc des intérêts multiples tant sur le plan galénique, toxicologique et économique que pour le confort du patient, en particulier s'il a été traité plusieurs fois aux dérivés imidazolés et si le traitement a engendré des souches résistantes à ces antifongiques.

Les compositions selon l'invention sont notamment utiles dans le traitement d'une affection choisie parmi les dermites séborrhéiques, les dermatoses eczématiques, le pityriasis versicolor, le psoriasis, les états squameux et pelliculaires.

Les exemples qui suivent sont destinés à illustrer certains aspects de l'invention.

Dans ces exemples, on se référera à la figure en annexe, qui représente l'isobologramme de l'association crotamiton/goudron 3 sur Pityrosporum ovale 1.

## Exemple 1

Des souches de Malassezia furfur (Pityrosporum ovale) ont été testées pour leur sensibilité vis-à-vis des principes actifs seuls ou en association.

Cette levure qui appartient à la flore cutanée humaine est représentative de la pathologie des états pelliculaires et squameux, puisqu'elle a été identifiée comme l'agent étiologique du pityriasis versicolor et une relation a été trouvé entre M. furfur et la dermatite séborrhéique (Shuster S. Brit. J. Dermatol., 1984, 11, 235-242). Les souches sont issues du Laboratoire de parasitologie du Centre Hospitalier Régional Rangueil de Toulouse (France) et une souche est éconazole-résistante (éco-R). Elles ont été cultivées lors des essais sur milieu Dixon liquide et repiquées et entretenues sur milieu Dixon gélosé.

La technique de l'échiquier consiste à réaliser une association complète de deux gammes de dilution sériées de deux antifongiques, de façon que chaque concentration d'un antifongique se trouve en présence de toutes les concentrations de la gamme de l'autre antifongique et inversement.

La méthode de l'échiquier a été réalisée en microplaques stériles Nunc R (microméthode) avec les témoins de stérilité de milieu, de croissance des germes et de valeurs des CMF validant chaque essai.

Après ensemencement à l'aide d'un multiensemenceur manuel de type Steers, les cultures sont incubées 4 jours à 32° C.

La lecture a lieu visuellement par rapport au témoin et le calcul des FIC a été fait à partir des concentrations obtenues.

Les produits testés sont :

- crotamiton : les solutions-mère sont réalisées extemporanément à 1% dans l'alcool à 60%,
- goudron 3 : coaltar crude rectifié ; la solution-mère à 2% est réalisée dans du Tween 20 à 20%.

Les résultats des essais sont résumés dans le tableau 1 ci-dessous. L'isobologramme de cette association est représenté sur la figure en annexe.

La mesure des FIC permet de conclure à la synergie, le meilleur FIC étant égal à 0,5.

**Tableau 1**

| | CMF seul | CMF associé | | | | | | |
|---|---|---|---|---|---|---|---|---|
| Crotamiton % | 0,031 | 0,0078 | 0,001 | 0,002 | 0,0039 | 0,015 | 0,0078 | 0,015 |
| Goudron 3% | 0,031 | 0,0078 | 0,015 | 0,015 | 0,015 | 0,003 | 0,015 | 0,0078 |
| FIC | | 0,5 | 0,53 | 0,56 | 0,62 | | 0,75 | |

**Exemple 2**

L'association du crotamiton avec deux autres types de goudron a été testée en suivant la méthodologie décrite à l'exemple 1.

On effectue des solutions-mère de coaltar à 2% dans de l'éthanol à 60° à partir de :

- goudron 1 = extrait végétal MF 1024 (pin, cade, bouleau),
- goudron 2 = coaltar USP (extrait alcoolique à 20% de coaltar crude).

Les synergies observées entre les goudrons et le crotamiton sont résumées dans le tableau 2 ci-dessous.

Tableau 2

| Goudron | Crotamiton | | |
|---|---|---|---|
| | 1 | 2 | 3 |
| Moyennes des FIC | 0,75<br>0,53 | 0,37<br>0,75 | 0,5<br>0,5 |

**Exemple 3**

**Exemples de formulations**

| **SHAMPOOING 1** | |
|---|---|
| COALTAR CRUDE | 0,5 % |
| CROTAMITON | 2 % |
| ACIDE SALICYLIQUE | 0,5 % |
| POLYSORBATE 20 | 8 % |
| COCOAMPHODIACETATE (30 %) | 10 % |
| ALKYLETHERSULFATE de Na (30 %) | 30 % |
| DISTEARATE DE PEG 6000 | 3 % |
| MONOSTEARATE D'ETHYLENE GLYCOL | 1,5 % |
| EAU PURIFIEE QSP | 100 ml |
| **SHAMPOOING 2** | |
| COALTAR SOLUTION USP | 2 ml |
| CROTAMITON | 2 g |
| COCOAMPHOGLYCINATE (30 %) | 1,5 g |
| LAURYL SULFATE D'AMMONIUM | 7 % |
| POLYSORBATE 20 | 5 % |
| ELFACOS GT 282 S | 2,8 % |
| PARFUM QS | |
| EAU PURIFIEE QSP | 100 ml |
| **SOLUTION CAPILLAIRE** | |
| COALTAR SOLUTION USP | 1,5 % |
| CROTAMITON | 1 % |
| ACIDE SALICYLIQUE | 0,25 % |
| D-PANTHENOL | 0,50 % |
| CREMOPHOR RH 40 | 3 % |
| ALCOOL ETHYLIQUE 95° | 63 % |
| EAU PURIFIEE QSP | 100 ml |

(suite)

| CREME CAPILLAIRE | |
|---|---|
| CROTAMITON | 3 % |
| COALTAR | 3 % |
| GOUDRON DE PIN | 0,30 % |
| GOUDRON DE CADE | 0,30 % |
| ACIDE SALICYLIQUE | 0,75 % |
| STEARYLDIMETHYLAMINE OXYDE | 2,5 % |
| POLYSORBATE 20 | 3 % |
| LANOLATE DE SORBITAN (40 EO) | 7,5 % |
| PARAFFINE PLAQUE | 5 % |
| LANOLINE | 3 % |
| PROPYLENE GLYCOL | 5 % |
| EAU PURIFIEE QSP | 100 % |
| **SOLUTION POUR LE BAIN** | |
| CROTAMITON | 3 % |
| COALTAR LAVE | 3 % |
| POLYSORBATE 20 | 20 % |
| CETIOL HE | 10 % |
| NONOXYNOL 9 | 10 % |
| PROPYLENE GLYCOL | 20 % |
| ETHOXYDIGLYCOL QSP | 100 g |
| **PAIN DERMATOLOGIQUE** | |
| COALTAR LAVE | 0,75 g |
| CROTAMITON | 1,50 g |
| ETHOXYDIGLYCOL | 0,75 g |
| PEG-75 LANOLIN ALCOHOL | 0,50 % |
| BASE ISETHIONATE/HEMISULFOSUCCINATE . QSP | 100 g |

## Revendications

1. Composition dermocosmétique, antifongique, **caractérisée en ce qu'**elle contient, outre un véhicule dermatologiquement acceptable, une association synergique de crotamiton et d'au moins un goudron, **à condition que ce goudron ne soit pas un goudron de soja**.

2. Composition selon la revendication 1, **caractérisée en ce que** le goudron est choisi parmi les dérivés de la houille, les dérivés végétaux issus de la pyrolyse des bois, les dérivés de schistes bitumeux, ainsi que leurs mélanges.

3. Composition selon la revendication 2, **caractérisée en ce qu'**au moins un goudron est choisi dans le groupe constitué par le goudron de pin, le goudron de cade, le goudron de bouleau et l'ichtyol ammonium.

4. Composition selon l'une des revendications 1 à 3, **caractérisée en ce que** le ou les goudrons est présent à une concentration comprise entre 0,5 et 5g/100 ml.

5. Composition selon l'une des revendications 1 à 4, **caractérisée en ce que** le crotamiton est présent à une concentration comprise entre 0,25 et 5 g/100 ml.

6. Composition selon l'une des revendications 1 à 5, **caractérisé en ce que** le rapport goudron(s)/crotamiton est compris entre 2 et 20.

7. Composition selon l'une des revendications 1 à 6, **caractérisée en ce que** le rapport goudron/crotamiton est

compris entre 1et 4.

8. Utilisation d'une association synergique de **crotamiton et d'au moins un goudron** pour la préparation d'une composition derma-cosmétique destinée au traitement d'une affection choisie parmi les dermites séborréhiques, les dermatoses eczématiques, le pityriasis versicolor, le psoriasis, les états squameux et pelliculaires.

9. **Utilisation selon la revendication 8, caractérisée en ce que l'association synergique de crotamiton et d'au moins un goudron se définit selon l'une quelconque des revendications 2 à 7**.

**Claims**

1. Fungicidal dermocosmetic composition, **characterized in that** it comprises, in addition to a dermatologically acceptable vehicle, a synergistic combination of crotamiton and of at least one tar, provided that this tar is not a soybean tar.

2. Composition according to Claim 1, **characterized in that** the tar is chosen from coal derivatives, plant derivatives resulting from the pyrolysis of wood, oil shale derivatives, and their mixtures.

3. Composition according to Claim 2, **characterized in that** at least one tar is chosen from the group consisting of pine tar, cade tar, birch tar and ammonium bituminosulphonate.

4. Composition according to one of Claims 1 to 3, **characterized in that** the tar or tars is present at a concentration of between 0.5 and 5 g/100 ml.

5. Composition according to one of Claims 1 to 4, **characterized in that** the crotamiton is present at a concentration of between 0.25 and 5 g/100 ml.

6. Composition according to one of Claims 1 to 5, **characterized in** the tar(s)/crotamiton ratio is between 2 and 20.

7. Composition according to one of Claims 1 to 6, **characterized in that** the tar/crotamiton ratio is between 1 and 4.

8. Use of a synergistic combination of crotamiton and of at least one tar for the preparation of a dermocosmetic composition intended for the treatment of an ailment chosen from seborrhoeic dermatitis, eczematous dermatosis, pityriasis versicolor, psoriasis, and scaly and dandruff conditions.

9. Use according to Claim 8, **characterized in that** the synergistic combination of crotamiton and of at least one tar is defined according to any one of Claims 2 to 7.

**Patentansprüche**

1. Dermokosmetische Antifungus-Zusammensetzung, **dadurch gekennzeichnet, dass** sie außer einem dermatologisch annehmbaren Träger eine synergistische Verbindung von Crotamiton und mindestens einem Teer enthält, unter der Bedingung, dass der Teer nicht ein Sojateer ist.

2. Zusammensetzung nach Anspruch 1, **dadurch gekennzeichnet, dass** der Teer ausgewählt ist aus Derivaten von Kohle, pflanzlichen Derivaten, die aus der Pyrolyse von Hölzern hervorgegangen sind, Derivaten von bituminösen Schiefern sowie deren Mischungen.

3. Zusammensetzung nach Anspruch 2, **dadurch gekennzeichnet, dass** mindestens ein Teer ausgewählt ist aus der Gruppe bestehend aus Kiefernteer, Wacholderteer, Birkenteer und Ammoniumichthyol.

4. Zusammensetzung nach einem der Ansprüche 1 bis 3, **dadurch gekennzeichnet, dass** die Teere bei einer Konzentration von 0,5 bis 5 g/100 ml vorliegen.

5. Zusammensetzung nach einem der Ansprüche 1 bis 4, **dadurch gekennzeichnet, dass** das Crotamiton bei einer Konzentration von 0,25 bis 5 g/100 ml vorliegt.

6. Zusammensetzung nach einem der Ansprüche 1 bis 5, **dadurch gekennzeichnet, dass** das Verhältnis Teer(e)/Crotamiton 2 bis 20 beträgt.

7. Zusammensetzung nach einem der Ansprüche 1 bis 6, **dadurch gekennzeichnet, dass** das Verhältnis Teer/Crotamiton 1 bis 4 beträgt.

8. Verwendung einer synergistischen Verbindung von Crotamiton und mindestens einem Teer für die Herstellung einer dermokosmetischen Zusammensetzung, die zur Behandlung eines Leidens bestimmt ist, das ausgewählt ist aus seborrhöischen Dermatitiden, ekzematischen Dermatosen, Pityriasis versicolor, Psoriasis, Schuppen- und Kopfschuppen-Zuständen.

9. Verwendung nach Anspruch 8, **dadurch gekennzeichnet, dass** die synergistische Verbindung von Crotamiton und mindestens einem Teer gemäß irgendeinem der Ansprüche 2 bis 7 definiert ist.

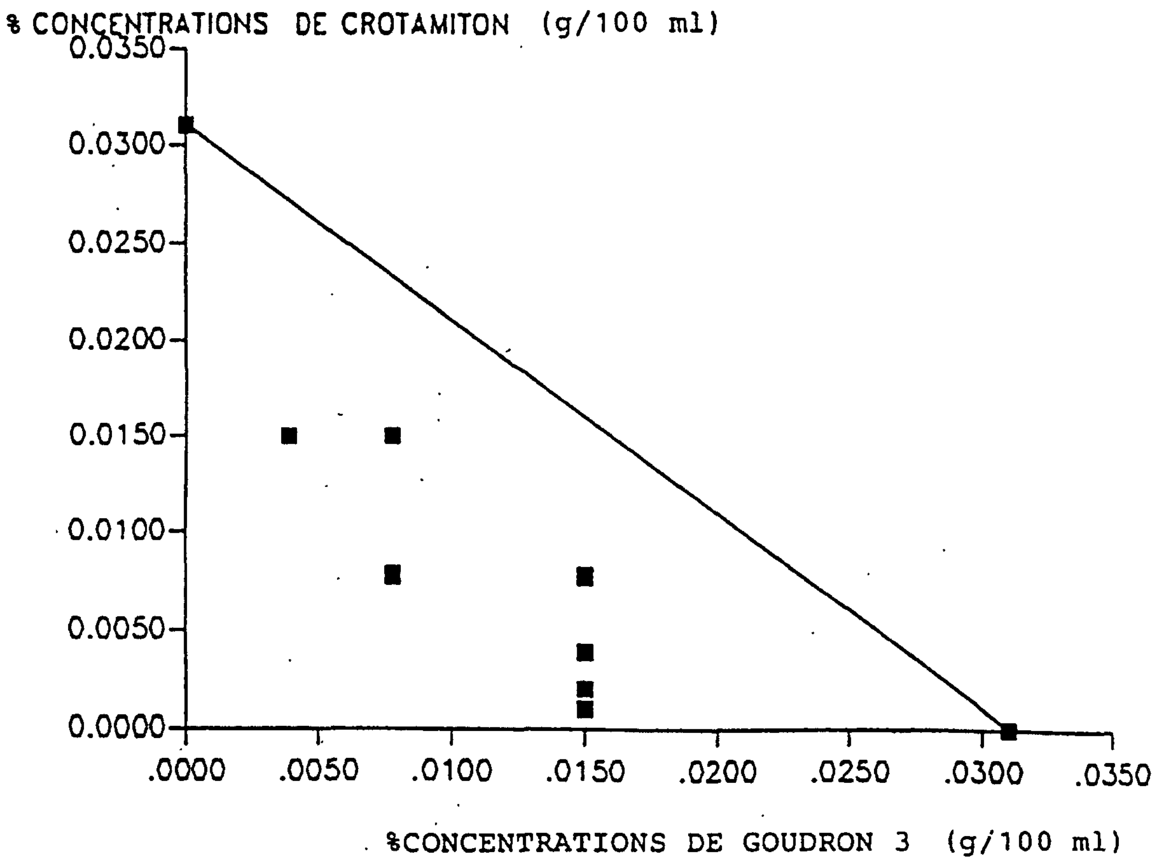
% CONCENTRATIONS DE CROTAMITON (g/100 ml)
0.0350
0.0300
0.0250
0.0200
0.0150
0.0100
0.0050
0.0000
.0000
.0050
.0100
.0150
.0200
.0250
.0300
.0350
%CONCENTRATIONS DE GOUDRON 3 (g/100 ml)

FIG.1